Europäisches Patentamt

European Patent Office

Office européen des brevets

(11) Veröffentlichungsnummer: **0 362 612**
**A1**

## EUROPÄISCHE PATENTANMELDUNG

(21) Anmeldenummer: **89117258.7**

(22) Anmeldetag: **18.09.89**

(51) Int. Cl.⁵ **B65F 7/00**

(30) Priorität: **30.09.88 DE 3833281**

(43) Veröffentlichungstag der Anmeldung:
**11.04.90 Patentblatt 90/15**

(84) Benannte Vertragsstaaten:
**AT BE CH ES FR GB GR IT LI NL SE**

(71) Anmelder: **NORDPUNKT AG**

**CH-6986 Novaggio/TI(CH)**

(72) Erfinder: **Maihofer, Willi**
**Villa Monna Lisa**
**CH-6986 Novaggio/TI(CH)**

(74) Vertreter: **Dipl.-Ing. Schwabe, Dr. Dr.**
**Sandmair, Dr. Marx**
**Stuntzstrasse 16**
**D-8000 München 80(DE)**

(54) **Hermetisch verschliessbarer Müllbehälter.**

(57) Die Erfindung betrifft einen hermetisch durch einen Deckel verschließbaren Müllbehälter, der für die Sterilisierung von in ihm enthaltenen Müll in einem Mikrowellen-Sterilisierungstunnel geeignet ist, wobei der Behälter und/oder der Deckel einen nach außen vorspringenden hohlen Handgriff aufweist, der als Wasserreservoir dient und der durch eine bei Wärmeeinwirkung sich auflösende oder aufreißende Folie verschlossen ist.

EP 0 362 612 A1

## Hermetisch verschließbarer Müllbehälter

Die Erfindung betrifft einen hermetisch verschließbaren Müllbehälter nach dem Oberbegriff des Anspruches 1.

In der DE 37 10 156 A1 ist eine Vorrichtung beschrieben, die zur Sterilisation von Krankenhausmüll mittels Mikrowellen dient. Diese Vorrichtung umfaßt eine Mikrowellenkammer mit einer Eingangstür und einer Ausgangstür, über die der Müll in die Mikrowellenkammer eingebracht und nach Sterilisation desselben wieder ausgetragen wird. Der Müll wird bei dieser bekannten Vorrichtung zunächst in hermetisch mit einem Deckel verschließbare Behälter abgefüllt und diese Behälter werden dann in das Mülleintragende der als Sterilisierungstunnel ausgebildeten Mikrowellenkammer über die genannte Eingangstür eingebracht. Der im Behälter untergebrachte Müll kann aus einem im oder am Behälter oder Behälterdeckel angeordneten Wasserreservoir befeuchtet werden, um dadurch die Sterilisierungswirkung zu erhöhen.

Aus der DE-OS 33 17 300 ist ein Behälter zur Aufnahme von Infektionsmüll, insbesondere von Krankenhausabfall bekannt.

Zur Verhütung von Infektionen wird dieser Müll mit Hilfe von Mikrowellen sterilisiert, was dadurch möglich ist, daß der von außen verschließbare Behälter aus einem Material besteht, durch welches Mikrowellen hindurch gelangen können. Dieser Behälter ist in seinem Inneren mit einem einsetzbaren, standfesten und verschließbaren Einmal-Innenbehälter für den Infektionsmüll ausgestattet, der aus einem im wesentlichen feuchtigkeitsdichten, sowie Dampf, jedoch Mikrowellen hindurchlassenden reißfesten Material besteht. Der Außenbehälter dieser Anordnung kann als Druckbehälter ausgeführt sein. Der Innenbehälter kann mit einem Deckel mit Schnappverschluß ausgestattet sein. An der Innenseite des Deckels des genannten Innenbehälters kann ein Beutel oder dergleichen mit Flüssigkeit, vorzugsweise Desinfektionsmittel angebracht sein, der durch die verdampfende Flüssigkeit im Inneren desselben zum Platzen gebracht wird, damit die Flüssigkeit in den Müll eindringen kann.

Aus der DE-OS 29 08 086 ist ein Verfahren und eine Vorrichtung zum Desinfizieren und Sterilisieren von mit Keimen behafteten Gegenständen bekannt. Das wesentliche dieses bekannten Verfahrens besteht darin, daß im Raum um den zu behandelnden Gegenstand ein Desinfektionsmittel vernebelt wird. Dies kann beispielsweise mit Hilfe von Druckluft durchgeführt werden. Zusätzlich kann dann der von dem Desinfektionsnebel umgebene Gegenstand mit Mikrowellen bestrahlt werden. Die Vorrichtung zur Durchführung dieses bekannten Verfahrens umfaßt eine abgedichtete Desinfektionskammer, wobei in der Desinfektionskammer ein Vernebelungsgerät für Desinfektionsmittel angeordnet ist.

Die der Erfindung zugrundeliegende Aufgabe besteht darin, einen hermetisch verschließbaren Müllbehälter der angegebenen Gattung zu schaffen, bei dem auf besonders einfache Weise Wasser in das Innere des Müllbehälters eingebracht werden kann, ohne daß der den Müll aufnehmende Innenraum des Müllbehälters in irgendeiner Weise eingeschränkt wird.

Diese Aufgabe wird erfindungsgemäß durch die im Kennzeichnungsteil des Anspruches 1 aufgeführten Merkmale gelöst.

Eine besonders vorteilhafte Ausgestaltung der Erfindung ergibt sich aus dem Anspruch 2.

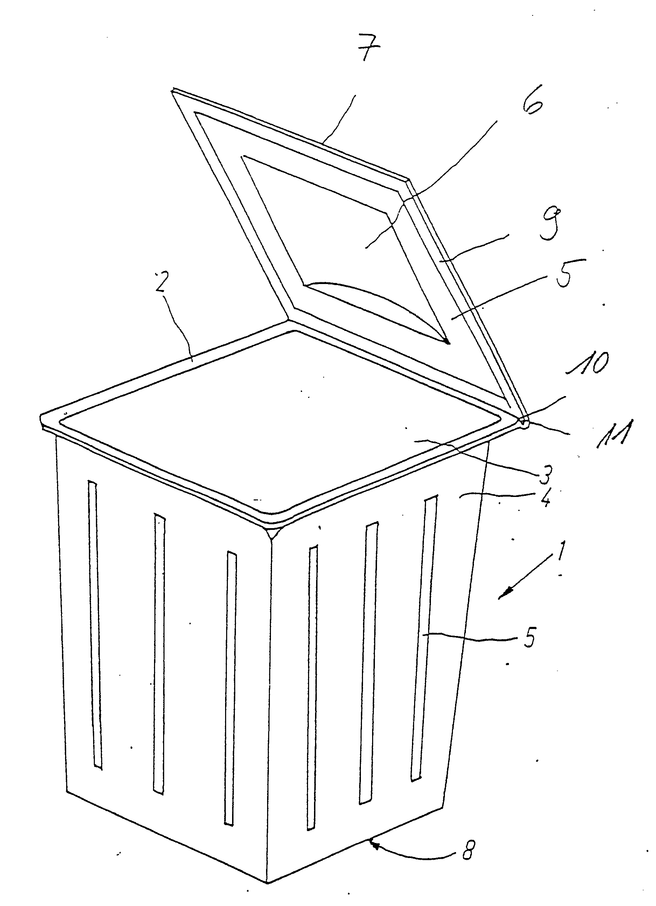

Im folgenden wird die Erfindung anhand eines Ausführungsbeispiels unter Hinweis auf die Zeichnung näher erläutert, deren einzige Fig. eine schematische perspektivische Ansicht eines Müllbehälters mit Deckel mit Merkmalen nach der Erfindung zeigt.

Die einzige Figur zeigt schematisch eine perspektivische Darstellung eines Müllbehälters, der allgemein mit 1 bezeichnet ist. Der gezeigte Müllbehälter hat eine quaderförmige Gestalt und besitzt Seitenwände, die durch Verstärkungsrippen 5 verstärkt sind. Am oberen Randbereich der Einfüllöffnung des Behälters ist ein Abdichtwulst 2 ausgebildet, wobei bei dem gezeigten Ausführungsbeispiel der obere Öffnungsquerschnitt des Behälters dem Querschnitt des Innenraums 3 des Behälters entspricht. An einem Längsrand des oberen Öffnungsrandes ist bei 10, 11 ein Deckel 5 angelenkt, der einen sich umfangsmäßig erstreckenden Abdichtflansch 9 und 7 aufweist. Im mittleren Bereich des Deckels ist eine kuppelförmige Ausnehmung 6 ausgebildet, die auf zwei gegenüberliegenden Seiten von zwei kreisabschnittförmigen Wandteilen begrenzt ist. Diese zwei sich gegenüberliegenden Wandabschnitt können als Griff verwendet werden, um den Deckel von außen anzuheben und zu öffnen. Der gezeigte Hohlraum 6 ist normalerweise mit Wasser gefüllt, wobei die gezeigte Öffnung mit einer geeigneten Folie oder einer Siegellackschicht verschlossen ist. Wenn der gezeigte Müllbehälter mit Müll gefüllt ist, so wird der Deckel 5 hermetisch an dem oberen Rand 2 des Behälters abgedichtet und verschlossen, woraufhin dann der Behälter zu einem Sterilisierungstunnel transportiert wird.

Wenn ein solcher Behälter einen ersten Mikrowellengenerator im Inneren eines Sterilisierungstunnel erreicht, so wird dadurch die Folie, welche das Wasserreservoir innerhalb des Behälters oder innerhalb des Deckels verschließt, erhitzt, wodurch sich die Folie auflöst oder zumindest aufreißt, so daß das Wasser in den Müll eindringen kann, wodurch die Sterilisierung und Desinfektion äußerst wirksam gestaltet werden kann.

Es besteht ferner die Möglichkeit, das Wasserreservoir nicht im Bereich des Deckels vorzusehen, sondern einen entsprechend hohlen Griff am Behälter selbst vorzusehen, der dann ebenfalls mit einer schmelzbaren Folie verschlossen wird, um den Wasservorrat während des Sterilisierungsvorganges freizugeben.

Es besteht ebenso die Möglichkeit, neben der Sterilisierung des Mülls mit Hilfe von Mikrowellen gleichzeitig chemische Mittel anzuwenden, d.h. der oder die Behälter zur Abgabe von Flüssigkeit können mit Behältern kombiniert werden, die ein Desinfektionsmittel enthalten oder es kann an die Stelle des Wassers auch nur ein Desinfektionsmittel treten.

Darüber hinaus besteht die Möglichkeit, unabhängig von dem Wasserreservoir im Deckel oder im Müllbehälter noch zusätzlich Behälter mit Wasser oder einem Desinfektionsmittel vor, während oder nach der Füllung der Behälter mit Müll mit ein zufüllen, wodurch sichergestellt wird, daß eine innige Durchtränkung des gesamten Mülls mit dem Wasser oder dem Desinfektionsmittel sichergestellt wird. Diese genannten Behälter können ebenfalls aus einem Material hergestellt sein, welches sich bei Einwirkung von Mikrowellenenergie auflöst oder zumindest aufreißt, um den flüssigen Inhalt freizugeben.

## Ansprüche

1. Hermetisch durch einen Deckel verschließbarer Müllbehälter, der für die Sterilisierung von in ihm enthaltenen Müll in einem Mikrowellen-Sterilisierungstunnel geeignet ist, dadurch **gekennzeichnet,** daß der Behälter und/oder der Deckel einen nach außen vorspringenden hohlen Handgriff aufweist, der als Wasserreservoir (6) dient und der durch eine bei Wärmeeinwirkung sich auflösende oder aufreißende Folie verschlossen ist.

2. Vorrichtung nach Anspruch 1, dadurch **gekennzeichnet,** daß die Folie aus einer Siegellackschicht besteht.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-3 505 892 (GÖLDNER) <br> * Figuren 1,3; Seite 4, Zeile 27 - Seite 5, Zeile 2; Seite 6, Zeilen 29-35 * | 1 | B 65 F 7/00 |
| Y | --- | 2 | |
| Y | US-A-2 917 116 (WYANT) <br> * Spalte 2, Zeilen 7-27,56-73; Figur 1 * | 2 | |
| D,A | DE-A-3 317 300 (VIEREGGE-BRUNS) <br> --- | | |
| A | EP-A-0 059 822 (PUCADLA) <br> ----- | | |

|  |
|---|
| RECHERCHIERTE SACHGEBIETE (Int. Cl.5) |
| B 65 F |

Der vorliegende Recherchenbericht wurde für alle Patentansprüche erstellt

| Recherchenort | Abschlußdatum der Recherche | Prüfer |
|---|---|---|
| DEN HAAG | 02-01-1990 | DEUTSCH J.P.M. |